(19) Europäisches Patentamt
European Patent Office
Office européen des brevets

(11) Veröffentlichungsnummer: **0 334 984 B1**

(12) **EUROPÄISCHE PATENTSCHRIFT**

(45) Veröffentlichungstag der Patentschrift: **03.06.92**

(51) Int. Cl.⁵: **C01B 33/148**, A61K 6/06

(21) Anmeldenummer: **88105248.4**

(22) Anmeldetag: **31.03.88**

# (54) Froststabilisiertes Kieselsol und seine Verwendung als Liquid für Einbettmassen auf Phosphatbasis.

(43) Veröffentlichungstag der Anmeldung:
**04.10.89 Patentblatt 89/40**

(45) Bekanntmachung des Hinweises auf die Patenterteilung:
**03.06.92 Patentblatt 92/23**

(84) Benannte Vertragsstaaten:
**AT BE CH DE ES FR GB GR IT LI LU NL SE**

(56) Entgegenhaltungen:
**DE-A- 1 542 905**
**US-A- 2 601 291**
**US-A- 4 057 525**

(73) Patentinhaber: **Giulini Chemie GmbH**
**Giulinistrasse 2 Postfach 150 480**
**W-6700 Ludwigshafen/Rhein(DE)**

(72) Erfinder: **Förster, H.-J., Dr. Dipl.-Chem.**
**Osterweg 1**
**W-6830 Schwetzingen(DE)**
Erfinder: **Gotzig, Josef, Dr. Dipl. Chem.**
**Albert Schweitzer Strasse 4**
**W-6701 Dannstadt-Schauernheim(DE)**
Erfinder: **Brenn, Jörg**
**Jahnstrasse 85**
**W-6832 Hockenheim(DE)**
Erfinder: **Tucholke, Wolfgang**
**Weilerhöferweg 8**
**W-6068 Riedstadt(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein froststabilisiertes Kieselsäuresol zur Verwendung als Liquid für Einbettmassen auf Phosphatbasis in der Dentaltechnik.

Einbettmassen sind in der Dentaltechnik schon seit langem bekannt. Sie werden zur Herstellung von metallischen Präzisionsgüssen eingesetzt und bestehen im wesentlichen aus Mischungen von Quarzmehl, Cristobalit und Tridymit sowie einem Bindemittel. Letzteres ist bei niedrig schmelzenden Metallen meistens ein Calciumsulfat-Halbhydrat und bei Metallen mit einem hohen Schmelzintervall ein Phosphatbinder, bestehend aus einem aktiven Magnesiumoxid oder aktiven Magnesiumhydroxid und Monoammoniumorthophosphat. Weitere Bestandteile der Einbettmassen können die Abbindegeschwindigkeit und -expansion beeinflussende Komponenten sein. Auch können sie noch andere Hilfstoffe, z.B. temperaturbeständige Füllstoffe und Graphit enthalten.

Bei Zugabe von Wasser und/oder handelsüblichem Kieselsol und späterem Erhitzen binden die Komponenten des Bindemittels gemäß den nachstehenden Gleichungen zu einer hochfeuerfesten Masse ab.

(1) $MgO + NH_4H_2PO_4 + 5\ H_2O \rightarrow NH_4MgPO_4 \cdot 6H_2O$

(2) $2NH_4MgPO_4 \cdot 6\ H_2O \rightarrow Mg_2P_2O_7 + 7H_2O\uparrow + 2\ NH_3\uparrow$

Es ist weiterhin bekannt, daß es bei der Abbindung der Einbettmassen zu erheblichen Volumenveränderungen kommt, die sich zusammensetzten aus:

1. Erstarrungs- oder Kälteexpansion (Kristallwasseraufnahme)
2. thermische Expansion (Expansion der Quarzmodifikationen, insbesondere des Cristobalits)
3. thermische Kontraktion (Abkühlung der erhitzten Einbettmasse).

Daß diese Volumenänderungen (Expansionsvermögen) durch Konzentrationsveränderungen im Kieselsäuresol gesteuert werden können, ist ebenfalls Stand der Technik. Handelsübliche Kieselsäuresole, die in wäßriger Lösung bis zu 60 Gew.% kolloidale Kieselsäure enthalten, werden daher in der Praxis als Liquid für Einbettmassen auf Phosphatbasis häufig verwendet.

Im Handel erhältliche Kieselsäuresole sind u.a beschrieben in der DE-OS 21 10 058. Bei dem dort beschriebenen Verfahren werden sie allerdings nicht als Liquid für Einbettmassen auf Phosphatbasis eingesetzt, sondern als Liquid (Anmachflüssigkeit) für Calciumsulfat-Halbhydrat. Diese Kieselsäuresole können durch Zugabe amphoterer Metallhydroxide stabilisiert sein, z.B. durch Hydroxide des Aluminiums, Zinns, Zinks oder Bleis. Bei Temperaturen um den Gefrierpunkt und darunter kommt es jedoch auch bei diesen Solen zu irreversiblen Ausfällungen. Ein nicht froststabilisiertes Kieselsäuresol bildet bereits bei einmaligem Frost-Tau-Wechsel ein in Wasser nur unvolständig lösliches Gel. Eine Redispergierung des kolloidalen $SiO_2$ tritt praktisch nicht ein.

Aus diesem Grunde ist auch bereits versucht worden, Kieselsäuresole mit bekannten oder neuen Gefrierschutzmitteln, das sind Substanzen, die den Gefrierpunkt herabsetzten, zu stabilisieren. So werden von einer Kieselsolherstellerin kieselsäuresole geliefert, die als "Gefrierschutzmittel" Monoethylenglykol enthalten. Die glykolhaltigen Sole sind zwar auch nach mehrmaligen Frost-Tau-Wechseln noch stabil, für Einbettmassen auf Phosphatbasis in der Dentaltechnik sind sie jedoch wegen ihrer ungünstigen Beeinflussung des Expansionsvermögens ungeeignet.

Die Froststabilisierung von Kieselsäuresolen, insbesondere Kieselsole enthaltenden Emulsionen von Wachs und Überzugsmitteln (Farbstoffmischungen), ist Gegenstand der US-PS 2,601,291. A. a. O. wird vorgeschlagen, Kieselsäuresole durch Zusatz van wasserlöslichen primären, sekundären oder tertiären Aminen froststabil zu machen.

Pro 100 g $SiO_2$ im Sol werden 0,05 bis 2,2 Mol Amin zugesetzt. Nach sechsmaligem Frost-Tau-Wechsel sollen sich lediglich Trübungen gezeigt haben, aber keine Ausfällungen. In Einbettmassen auf Phosphatbasis auf dem Gebiet der Dentaltechnik werden auch die vorstehenden Kieselsole nicht eingesetzt und als ungeeignet abgelehnt, weil das Expansionsverhalten der Einbettmassen nachteilig beeinflußt wird.

Es ist weiterhin gemäß DE-A-1 542 905 Kieselsäuresol bekannt, das einen $SiO_2$- Gehalt von > 6 Gew % aufweist und als Dispergierhilfsmittel Harnstoff enthält. Das gennante Kieselsäuresol wird als Bodenverbesserungsmittel verwendet.

Es stellte sich somit die Aufgabe, ein froststabiliziertes Kieselsäuresol zu finden, das auch nach wiederholtem Frost-Tau-Wechsel in Solform vorliegt, praktisch also keine Veränderungen zeigt, und das darüberhinaus als Liquid in Einbettmassen das Expansionsverhalten der Einbettmassen nicht negativ beeinflußt.

Überraschenderweise kann die gestellte Aufgabe mit einem Kieselsäuresol gelöst werden, das 25 bis

50 Gew.% $SiO_2$ und 2 - 10 Gew.% einer Verbindung der allgemeinen Formel

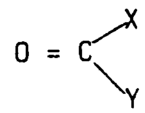

in der X und Y eine $NH_2$- oder NHR-Gruppe sein kann, und R einen Alkylrest mit 1 bis 4 Kohlenstoffatomen bedeutet, enthält. Besonders geeignet sind Kieselsäuresole mit $SiO_2$-Konzentrationen von 35 bis 45 Gew.% und Harnstoff- und/oder Harnstoffderivat-Konzentrationen von 5 bis 7 Gew.%. Der Einsatz von Kieselsäuresolen mit einer spezifischen Oberfläche nach BET von 150 bis 250 $m^2/g$, insbesondere 180 bis 210 $m^2/g$ ist dabei von Vorteil.

Besonders gute Ergebnisse werden mit Harnstoff und N-Methylharnstoff erhalten.

Der Harnstoff und/oder die Harnstoffderivate werden dem Kieselsäuresol zweckmäßigerweise in fester Form zugesetzt, und zwar bei Raumtemperatur. Sie werden unter Rühren gelöst. Sie können gegebenenfalls auch in wäßriger Lösung zugesetzt werden.

Die erfindungsgemäßen Kieselsäuresole können ohne Beeinträchtigung ihrer Konsistenz einem wiederholten Frost-Tau-Wechsel unterworfen werden.

Bei Zusatz von Harnstoff kann beispielsweise durch Frost-Tau-Wechsel (15 - 20mal) keine Destabilisierung mehr beobachtet werden. Eine zeitliche Änderung dieser Wirkung wird nicht beobachtet.

Als Liquid für Einbettmassen sind in besonderem Maße die Harnstoff enthaltenden Kieselsäuresole geeignet, da sie die Abbindeexpansion der Einbettmassen, verglichen mit der Abbindeexpansion von harnstofffreien Einbettmassen, nicht oder nur sehr wenig reduzieren. Sie können in gleicher Weise wie die nicht froststabilisierten Kieselsäuresole zur Expansionssteuerung der Einbettmassen eingesetzt werden, da auch ihre anwendungstechnischen Eigenschaften nicht beeinflußt werden.

Die Verwendung von Harnstoff als Auftaustoff auf Flughäfen sowie im Straßenwinterdienst ist zwar bekannt, jedoch konnte daraus die froststabilisierende Wirkung in Kieselsäuresolen nicht hergeleitet werden. Dies insbesondere auch deshalb nicht, weil andere Gefrierschutzmittel und Auftaumittel in handelsüblichen Kieselsäuresolen keine zufriedenstellende Wirkung zeigen, beispielsweise Glykole.

Anhand der nachstehenden Beispiele wird der Erfindungsgegenstand noch näher erläutert:

**Beispiele:**

In einem Kieselsäuresol mit einer $SiO_2$-Konzentration von 40 Gew.% und einer spezifischen Oberfläche nach BET von ca. 200 $m^2/g$ wurden bei Raumtemperatur (21° C ± 2° C) jeweils 6 Gew.% der folgenden Zusätze gelöst:

1. Harnstoff
2. N-Methylharnstoff
3. N-Ethylharnstoff
4. N,N'-Dimethylharnstoff

Die Proben wurden zusammen mit einer Probe des reinen Kieselsols in einer Kühltruhe 20 Stunden lang auf - 15° C abgekühlt, wobei alle Proben fest wurden. Nach Erwärmen auf Raumtemperatur bildet das Kieselsol ohne Zusatz eine farblose, undurchsichtige und gelartige Masse. Die Gemische, d.h. Kieselsol mit Zusatz, liegen dagegen im Vergleich zu den ursprünglichen, nicht gefrorenen Proben, unverändert vor. Dieser Frost-Tau-Wechsel wurde so oft wiederholt, bis in den Gemischen ein gelartiger Rückstand zu beobachten war. Folgende Ergebnisse wurden erhalten:

| | |
|---|---|
| Harnstoff: | nach 20 Frost-Tau-Wechseln noch ohne Rückstand |
| N-Methylharnstoff: | nach 4 Frost-Tau-Wechseln |
| N-Ethylharnstoff: | nach 5 Frost-Tau-Wechseln |
| N,N'-Dimethylharnstoff: | nach 6 Frost-Tau-Wechseln |

**Vergleichsbeispiele:**

100 g der Einbettmasse Gilvest EHT (Cristobalit/Quarz/MgO - Gemisch) wurden jeweils mit 40 Gew.%igem Kieselsol mit einer spezifischen Oberfläche nach BET von etwa 200 $m^2/g$ vermischt, und zwar:

a) mit Kieselsol ohne Froststabilisator (Bayer Kieselsol 200),

b) mit froststabilsiertem Kieselsol gemäß Stand der Technik (Bayer Kieselsol 200 S mit 5 Gew.% Monoethylenglykol bzw. 4 % Monoethylenglykol),

c) mit froststabilsiertem Kieselsol der Erfindung (6 Gew.% Harnstoff).

Die Messung der Abbindeexpansion erfolgt nach DIN 13 911 bei Raumtemperatur (21° C ± 2° C) im Extensometer mit einem Trog vom 100 mm Länge. Es wurden die Expansionen nach 1 Stunde und nach 2 Stunden gemessen.

**Tabelle**

| | a) | b) | | c) |
|---|---|---|---|---|
| $1^h$ | 2,36 | 1,21 | 1,09 | 2,09 |
| $2^h$ | 2,50 | 1,29 | 1,16 | 2,15 |

Die Meßwerte der Tabelle zeigen, daß das Expansionsverhalten der Einbettmassen von dem erfindungsgemäß stabilisierten Kieselsol nur geringfügig beeinflußt wird, die Arbeiten in der Dentaltechnik dadurch also wesentlich erleichtert und verbessert werden.

**Patentansprüche**

1. Froststabilisiertes Kieselsäuresol zur Herstellung von Einbettmassen auf Phosphatbasis in der Dentaltechnik, dadurch gekennzeichnet, daß es 25 bis 50 Gew.% SiO2 und 2 bis 10 Gew.%, insbesondere 5 - 7 Gew.%, einer Verbindung der allgemeinen Formel enthält:

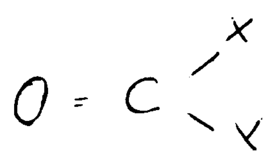

und in der X und Y eine NH2 oder NHR-Gruppe ist und R einen Alkylrest mit 1 bis 4 Kohlenstoffatomen bedeutet.

2. Froststabilisiertes Kieselsäuresol nach Anspruch 1, dadurch gekennzeichnet, daß es 5 bis 7 Gew.% Harnstoff enthält.

3. Froststabilisiertes Kieselsäuresol nach den Ansprüchen 1 und 2, dadurch gekennzeichnet, daß es 35 bis 45 Gew.% Siliciumdioxid enthält.

4. Froststabilisiertes Kieselsäuresol nach den Ansprüchen 1 bis 3, dadurch gekennzeichnet, daß es N-Methylharnstoff enthält.

5. Froststabilisiertes Kieselsäuresol nach den Ansprüchen 1 bis 4, dadurch gekennzeichnet, daß es N-Ethylharnstoff enthält.

6. Froststabilisiertes Kieselsäuresol nach den Ansprüchen 1 bis 5, dadurch gekennzeichnet, daß es N,N'-Dimethylharnstoff enthält.

## Claims

1. Silica sol stabilised against freezing for preparing embedding compounds based on phosphate in dental technology, characterised in that it contains 25 to 50 wt.% of $SiO_2$ and 2 to 10 wt.%, more particularly 5 to 7 wt.% of a compound in the general formula:

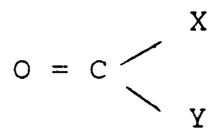

and wherein X and Y are $NH_2$ or NHR groups and R an alkyl group having 1 to 4 carbon atoms.

2. Silica sol stabilised against freezing according to claim 1, characterised in that it contains 5 to 7 wt.% of urea.

3. Silica sol stabilised against freezing according to claims 1 and 2, characterised in that it contains 35 to 45 wt.% of silicon dioxide.

4. Silica sol stabilised against freezing according to claims 1 to 3, characterised in that it contains N-methyl urea.

5. Silica sol stabilised against freezing according to claims 1 to 4, characterised in that it contains N-ethyl urea.

6. Silica sol stabilised against freezing according to claims 1 to 5, characterised in that it contains N,N'-dimethyl urea.

## Revendications

1. Sol d'acide silicique stabilisé contre la gelée pour la fabrication de masses d'enrobage à base de phosphate pour la technique dentaire, caractérisé en ce qu'il contient 25 à 50% en poids de SiO2 et 2 à 105 en poids, en particulier 5 à 7% en poids d'un composé de formule générale:

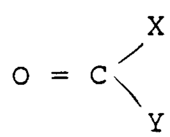

et que X et Y contiennent un groupe NH2 ou NHR, R étant un reste alkyle avec 1 à 4 atomes de carbone.

2. Sol d'acide silicique stabilisé contre la gelée selon la revendication 1, caractérisé en ce qu'il contient 5 à 7% en poids d'urée.

3. Sol d'acide silicique stabilisé contre la gelée selon les revendications 1 et 2, caractérisé en ce qu'il contient 35 à 45% en poids de dioxyde de silicium.

4. Sol d'acide silicique stabilisé contre la gelée selon les revendications 1 à 3, caractérisé en ce qu'il contient de la N-méthylurée.

5. Sol d'acide silicique stabilisé contre la gelée selon les revendications 1 à 4, caractérisé en ce qu'il contient de la N-éthylurée.

6. Sol d'acide silicique stabilisé contre la gelée selon les revendications 1 à 5, caractérisé en ce qu'il contient de la N,N'-diméthylurée.